# EUROPEAN PATENT SPECIFICATION

(11) **EP 0 869 744 B1**
(45) Date of publication and mention of the grant of the patent: **13.09.2000**
(21) Application number: 96943279.8
(22) Date of filing: 20.12.1996
(51) Int. Cl.: A61B 17/80, A61M 37/00

(54) **AN ORHTOPAEDIC PROSTHETIC DEVICE**
ORTHOPÄDISCHE PROTHESEVORRICHTUNG
PROTHESE ORTHOPEDIQUE

(30) Priority: 22.12.1995 IT BO950603
(43) Date of publication of application: 14.10.1998
(73) Proprietor: Istituti Ortopedici Rizzoli, 40136 Bologna (IT)
(72) Inventor: GIARDINO, Roberto, I-40141 Bologna (IT); FINI, Milena, I-40068 San Lazzaro di Savena (IT)
(74) Representative: Lanzoni, Luciano
(86) International application number: IT9600258
(87) International publication number: WO9723171

(56) References cited:
- EP-A- 0 295 041
- WO-A-95/20368
- DE-A- 4 118 201
- DE-A- 4 313 192
- FR-A- 2 290 881

## Description

### Technical Field

The present invention relates to an orthopaedic prosthetic device which may be used in the treatment of comminuted and localised fractures, or when a portion of skeletal segment is removed during surgery, for example, during the removal of a bone tumour.

In particular, the present invention relates to an orthopaedic prosthetic device shaped and structured so as to allow, guide and facilitate the repair of the skeletal segment damaged, in those cases in which there is a loss of bone material between the two stumps of the segment which prevents the correct recomposition of the bone segment and the drawing together and contact of the ends of the two stumps.

### Background Art

Many osteosynthesis techniques are currently known in the orthopaedics sector which, through the use of auxiliary synthesis devices called internal or external fixators, tend to allow the repair of fractures and so the regeneration of the callus. As regards external fixators, there are devices which Wallow more rapid cohesion of the callus forming in the damaged zone, and rapid readjustment to the load upon the bone itself, since they are able to keep the anatomical axis of the damaged skeletal segment aligned as it heals. Moreover, in those cases in which a portion of bone material is missing from the distal and proximal stumps of the damaged skeletal segment, so that they are distanced from one another, internal fixators may be used, normally consisting of metal plates fixed to the bone stumps by suitable fixing means, which can only contain from the structural viewpoint of the connections, graft or pieces of bone from the bone bank. If the fracture is not complete, or if there is no loss of bone material, intermedullary pins are normally used, applied along the anatomical axis of the fractured bone.

Obviously, these means of synthesis are intended to create structures with mechanical specifications for the support and rigid blocking of the stumps of the damaged skeletal segment, said means being able to guarantee the correct position of the stumps as they heal. In other words, these means of synthesis are mechanical structures, either temporary, which are removed once the skeletal segment has healed, or permanent, which from the biomechanical load-bearing viewpoint, substitute the bone structure of the skeletal segment to which they are applied, as well as making use of the bone induction and bone conduction of the callus during the healing stage.

In all cases involving the use of these means of synthesis, the damaged skeletal segment regenerates spontaneously, thus the means of synthesis are only structural mechanical means able to facilitate the mobility of the patient, but do not directly affect the regeneration of the callus of the skeletal segment, and so are distinct from the callus which regenerates spontaneously.

DE 41 18 201 discloses a device for connecting fractured bone stumps, according to the preamble of present claim 1. This prior document does not relate to an orthopaedic prosthetic device for repairing a skeletal segment damaged in those cases in which there is a loss of bone material between the two stumps of the segment.

### Disclosure of the Invention

The object of the present invention is to provide an orthopaedic prosthetic device which may be applied at two stumps of a skeletal segment, creating a chamber for the regeneration of the callus which guarantees a more rapid growth of the callus and reduces the time required for repair of the damaged part, which can both bind and directly amalgamate with the callus regeneration process and co-operate, where necessary, with the said internal and external fixators.

The present invention relates to an orthopaedic prosthetic device, in particular applicable to at least two bone stumps, proximal and distal, deriving from a fracture of a skeletal segment, with technical features according to present claim 1.

The present invention is described below with reference to the accompanying drawings, which illustrate a preferred embodiment, and in which:
Figure 1 is a schematic front view of the orthopaedic prosthetic device disclosed, applied to two long bone segments and in combination with a conventional means of synthesis, such as an external fixator;
figure 2 is a perspective view of an alternative embodiment of the device shown in figure 1;
figures 3 to 4 show alternative embodiments of the device shown in the previous figures.

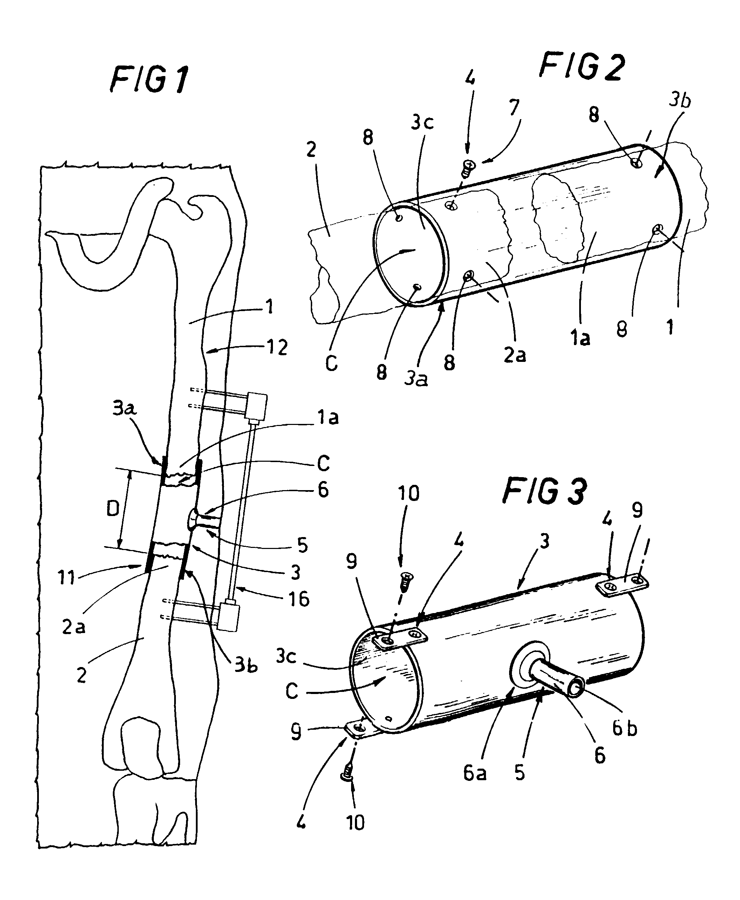

With reference to the accompanying drawings, the orthopaedic prosthetic device which is the subject matter of the present invention is indicated as a whole by the number 11. In figure 1, the prosthetic device 11 is shown applied to a skeletal segment 12 affected by a comminuted complete fracture or which has undergone surgery involving the removal of a portion of the segment 12 itself, for example removal of a tumour, and so has suffered a loss of bone material. The numbers 1 and 2 are used to indicate a pair of bone stumps, proximal 1 and distal 2, positioned at a distance D from one another, deriving from the afore-mentioned loss of bone material, their respective heads 1a and 2a being opposite one another.

Again in figure 1, the device 11 is in combination with a conventional means of synthesis 16, for example, an external fixator.

The distance D between the bone segments 1 and 2 is a function of the size of the comminuted complete fracture suffered, or of the portion of bone affected by a tumour removed.

According to the present invention, the device 11 comprises a tube 3 made of biocompatible, bioresorbable material, which is inserted between the said pair of bone stumps 1 and 2. The ends 3a and 3b of the tube 3 are attached to part of the two bone stumps 1 and 2, thus defining a chamber C, for protection, containment, guidance and regeneration, within which a callus can regenerate to rejoin the two bone stumps 1 and 2.

The tube 3 may have the shape of a cylinder or truncated cone, depending on the damaged bone zones, that is to say, it attempts to copy the development profile of the bone according to the part concerned.

As already indicated above, the tube 3 is used only to define a chamber C for regeneration of the callus, is made of various biocompatible, bioresorbable materials and has new applications in the orthopaedics sector, by which the chamber C binds with the callus which regenerates inside it.

In a first embodiment, the tube 3 may be made of biocompatible and bioresorbable material. Therefore, in this case the tube 3 constitutes a chamber C for regeneration of the callus, which is able to guide and correctly contain such growth of the skeletal segment 12, and at the same time be completely resorbed by the human body.

More specifically, the tube 3 is made of a material with special chemical and physical properties which render it compatible with the area surrounding bone segments 1 and 2, and also allow a controlled resorption over time, depending on the time required for the generation of the said callus between the two bone segments 1 and 2. In other words, it must be possible for the material used for the tube 3 to be resorbed within the body in a time practically equal to the time necessary for generation of the callus.

To obtain these properties, the tube 3 is made in various sizes and preferably of a polymer: for this purpose, in medical fields polylactic materials such as PLA, PLLA or PGA are known, as well as polyhydroxybutyrate or polycaprolactone (all biocompatible materials which are resorbed by the human body).

As shown in figure 2, the tube 3 has end fixing means 4 which fix the tube 3 between the two bone segments 1 and 2 at the respective heads 1a and 2a: this effectively defines the sealed chamber C for bone protection and regeneration between the two opposite heads 1a and 2a.

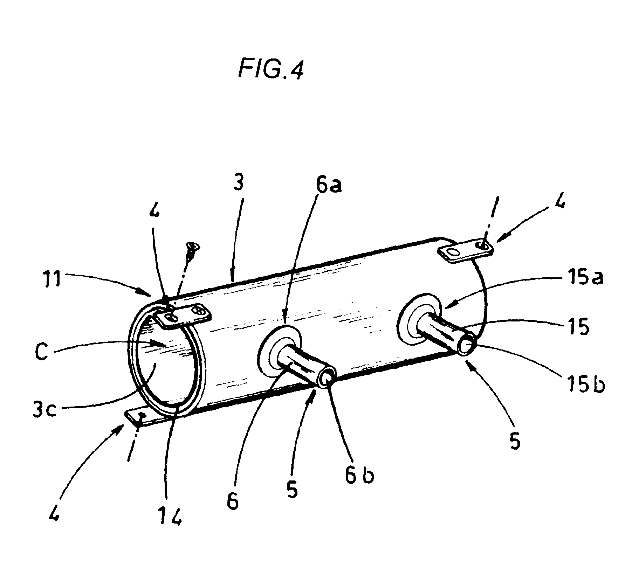

These fixing means 4 may be screws 7 which can be inserted in purpose made through-holes 8 made in the ends of the tube 3 (see figure 2) and screwed directly onto the heads 1a and 2a of the two bone segments 1 and 2. Similarly, small plates 9 may be used (see figure 3), fixed to the tube 3 and the ends of the bone segments 1 and 2, again using screws 10: in both cases, all elements which constitute the fixing means 4 are made of biocompatible and bioresorbable material, like the tube 3.

In another embodiment, the tube 3 may be made of a material with bone integrating properties, for example, a ceramic material.

This type of material, which may be either impermeable or porous, has chemical and physical properties which allow the tube 3 to amalgamate and compact itself with the callus which is generated inside the chamber C between the heads 1a and 2a of the stumps 1 and 2, forming a single body with the callus itself, which resorbs the tube 3. In other words, in this case the tube 3 constitutes a base with bone conduction and bone induction properties, which can form a single seamless body with the skeletal segment 12 to which it was applied.

In the embodiments illustrated in figures 1, 3 and 4, the tube 3 has respective inlet and/or outlet means 5 allowing communication between the chamber C and the outside. The means 5 applied to the tube 3, allow probes or suitable surgical apparatus to be inserted in the chamber C so as to check, sample and disinfect the internal zone of the chamber C, or allow the introduction of liquids intended to increase the bone growth factor.

These outlet and/or inlet means 5 may consist of a single conduit 6 or catheter (figure 3) made of biocompatible material, one end 6a of which leads into the tube 3, whilst the other end 6b leads directly to the outside of the skeletal segment upon which the tube 3 is fixed; here the catheter 6 may be fitted with safety means (e.g.: a non-return valve, not illustrated here) which prevent the accidental entrance of foreign bodies into the chamber C. The embodiment with at least one pair of conduits 6 and 15 (figure 4) is recommended for disinfecting the inside of the chamber C: one of the catheters is used to introduce sterilising agents, whilst the other allows the "dirty" liquid to be drawn out of the chamber C, so that one of the catheters is always completely "aseptic", for the introduction of any pharmaceuticals subsequently required.

The embodiment illustrated in figure 4 envisages that the internal surface 3c of the tube 3, that is to say, the surface which defines the chamber C, is lined with a layer of biocompatible and bioresorbable material, labelled 14, which can be loaded with slow-release substances which facilitate the reproduction of the callus inside the chamber C. The layer 14 of biocompatible and bioresorbable material may also be used to form sites for cell cultures, such as bone marrow cells and osteoblasts.

This embodiment, therefore, has significant advantages, one of which is the method of application, which envisages only one surgical operation, that involving connection of the device, thanks to the bone integration and/or bioresorbable nature of the entire device. Moreover, the idea of isolating the bone regeneration zone within a chamber C, which in some cases is substantially sealed, allows the following to be obtained: greater control and acceleration of the growth of the callus; maintenance of an aseptic environment, with the possibility of introducing both liquid sterilising agents and agents which promote bone growth, thanks to the conduits connected to the chamber C.

In some special cases, preferably during application of the tube 3, bone splinters may be inserted in the chamber C, further increasing the bone regeneration process, without these splinters being dispersed.

Isolation of the chamber C and all of the agents which it contains allows, on the whole, a considerable increase in the efficiency of the liquids and bone splinters in their bone regenerating role.

The present invention may be subject to numerous modifications and variations, all of which are encompassed by the design concept.

## Claims

1. An orthopaedic prosthetic device, in particular applicable to at least two bone stumps, proximal (1) and distal (2), deriving from a fracture of a skeletal segment (12), said stumps (1, 2) being positioned with the respective heads (1a, 2a) opposite one another wherein the device comprises a tube (3) made of biocompatible and bioresorbable material which may be inserted between the said pair of bone stumps (1, 2), containing, at the two opposite ends (3a, 3b), parts of the heads (1a, 2a) of the stumps (1, 2), and having means (4) made of biocompatible and bioresorbable material for fixing to the heads (1a, 2a) of the bone stumps (1, 2);
characterised in that said stumps (1, 2) are positioned with the respective heads (1a, 2a) at a distance (D) deriving from a loss of bone material from the said skeletal segment (12) which prevents the correct repositioning of the said segment (12) by drawing together and creating a substantial contact between the heads (1a, 2a) of the relative stumps (1, 2), said fixing means (4) being arranged at the ends (3a, 3b) of the tube (3), and in that the tube (3) is shaped and structured so as to define, at least for the entire distance (D) separating the two stumps (1, 2), a chamber (C) for the protection, containment, guidance and regeneration of the callus of the heads (1a, 2a) of the said stumps (1, 2) of the skeletal segment (12), designed to bind with the callus and become an integral part of it, and in that the wall of the tube (3) is continuous on its periphery except at the through-holes (8) and the outlet and/or inlet means (5).

2. The device as described in claim 1, characterised in that the tube (3) is made of a bioresorbable material with physical and chemical properties which guarantee a controlled resorption over time, depending on the time necessary for regeneration of the callus between the two bone stumps (1, 2).

3. The device as described in claim 1, characterised in that the tube (3) has at least means (5) for connecting the said chamber (C) to the outside environment, for introduction into the chamber (C) of pharmaceuticals and/or biological substances.

4. The device as described in claim 3, characterised in that the said means (5) for connection to the outside environment consist of at least one conduit (6) or catheter, one end (6a) of which leads into the tube (3), and the other end (6b) leading to the outside of the skeletal segment (12).

5. The device as described in claim 3, characterised in that the means (5) for connecting the chamber (C) to the outside environment include a further outlet conduit (15), one end (15a) of which leads into the tube (3), the other end (15b) leading to the outside of the skeletal segment (12).

6. The device as described in claim 1, characterised in that the tube (3) is preferably made of a polymer.

7. The device as described in claim 1, characterised in that the tube (3) is made of a biocompatible material, said material having physical and chemical bone integration properties, and being designed to amalgamate and compact itself with the callus generated between the two heads (1a, 2a) of the bone stumps (1, 2).

8. The device as described in claim 7, characterised in that the said material with bone integration properties is a bioceramic material.

9. The device as described in claim 8, characterised in that the said bioceramic material is a porous and permeable material.

10. The device as described in any of the previous claims, characterised in that the tube (3) consists of at least two longitudinal halves (17, 18), each having connecting means (13), said halves being biocompatible and designed to guarantee stable assembly of the tube (3).

11. The device as described in any of the previous claims, characterised in that the internal surface (3c) of the tube (3) delimiting the chamber (C) is lined by a layer (14) of biocompatible and bioresorbable material which may be loaded with slow-release substances that facilitate the reproduction of the callus.

12. The device as described in any of the previous claims, characterised in that the internal surface (3c) of the tube (3) delimiting the chamber (C) is lined by a layer (14) of biocompatible and bioresorbable material, said layer (14) being designed to form the site of cell cultures, such as bone marrow cells and osteoblasts which promote the reproduction of the callus.

13. The device as described in any of the previous claims, characterised in that the tube (3) has the shape of a cylinder or truncated cone.

14. The device as described in any of the previous claims, characterised in that the tube (3) defines a chamber (C), said chamber (C) being substantially sealed.

## Patentansprüche

1. Orthopädische Prothesenvorrichtung, insbesondere anbringbar an wenigstens zwei Knochenstümpfen, und zwar proximal (1) und distal (2), die sich aus einem Bruch eines Skelettsegmentes (12) ergeben, wobei die genannten Stümpfe (1, 2) sich mit ihren jeweiligen Kopfenden (1a, 2a) einander gegenüberliegend angeordnet befinden, und wobei die Vorrichtung ein Rohr (3) enthält, hergestellt aus einem körperverträglichen und bio-resorbierbaren Material, welches Rohr zwischen dem genannten Paar von Knochenstümpfen (1, 2) eingesetzt werden kann und an den beiden entgegengesetzten Enden (3a, 3b) Teile der Kopfenden (1a, 2a) der Stümpfe (1, 2) aufnimmt, und welches Mittel (4) aus körperverträglichem und bio-resorbierbaren Material zur Befestigung desselben an den Kopfenden (1a, 2a) hat; **dadurch gekennzeichnet,** dass die genannten Stümpfe (1, 2) mit ihren jeweiligen Kopfenden (1a, 2a) in einem Abstand (D) voneinander angeordnet sind, der sich aus einem Verlust von Knochenmaterie aus dem genannten Skelettsegment (12) ergibt, welcher durch ein Zusammenziehen und das Bilden eines wesentlichen Kontaktes zwischen den Kopfenden (1a, 2a) der jeweiligen Stümpfe (1, 2) die korrekte Repositionierung des genannten Segmentes verhindern würde, wobei die genannten Befestigungsmittel (4) an den Enden (3a, 3b) des Rohres (3) angeordnet sind, und dadurch, dass das Rohr (3) so ausgebildet und strukturiert ist, dass es wenigstens über den gesamten, die beiden Stümpfe (1, 2) trennenden Abstand (D) eine Kammer (C) beschreibt, die zum Schutz, zur Aufnahme, zum Formen und Regenerieren des Kallus der Kopfenden (1a, 2a) der genannten Stümpfe (1, 2) des Skelettsegmentes (12) dient, dazu bestimmt, sich mit dem Kallus zu verbinden und ergänzender Teil desselben zu werden, und dadurch, dass die Wand des Rohres (3) an ihrem Umfang durchgehend ist, mit Ausnahme an den durchgehenden Bohrungen (8) und an den Auslass- und/oder Einlassmitteln (5).

2. Vorrichtung nach Patentanspruch 1, **dadurch gekennzeichnet,** dass das Rohr (3) aus einem bio-resorbierbaren Material mit solchen physischen und chemischen Eigenschaften hergestellt ist, welche eine zeitlich gesteuerte Resorption gewährleistet, die von der Dauer abhängt, welche zur Regeneration des Kallus zwischen den beiden Knochenstümpfen (1, 2) notwendig ist.

3. Vorrichtung nach Patentanspruch 1, **dadurch gekennzeichnet,** dass das Rohr (3) wenigstens ein Mittel (5) zur Verbindung der genannten Kammer (C) mit dem Aussenbereich aufweist, um in die Kammer (C) pharmazeutische und/oder biologische Substanzen einführen zu können.

4. Vorrichtung nach Patentanspruch 3, **dadurch gekennzeichnet,** dass die genannten Mittel (5) zur Verbindung mit dem Aussenbereich aus wenigstens einer Leitung (6) oder einem Katheter bestehen, dessen eines Ende (6a) in das Rohr (3) führt, und dessen anderes Ende (6b) aus dem Skelettsegment (12) heraus führt.

5. Vorrichtung nach Patentanspruch 3, **dadurch gekennzeichnet,** dass die Mittel (5) zur Verbindung der Kammer (C) mit dem Aussenbereich eine weitere Auslasskanüle (15) enthält, deren eines Ende (15a) in das Rohr (3) führt, und deren anderes Ende (15b) aus dem Skelettsegment (12) heraus führt.

6. Vorrichtung nach Patentanspruch 1, **dadurch gekennzeichnet,** dass das Rohr (3) vorzugsweise aus Polymer hergestellt ist.

7. Vorrichtung nach Patentanspruch 1, **dadurch gekennzeichnet,** dass das Rohr (3) aus einem körperverträglichen Material hergestellt ist, wobei das genannte Material physische und chemische knochenintegrierende Eigenschaften aufweist und dazu dient, sich mit dem zwischen den beiden Kopfenden (1a, 2a) der Knochenstümpfe (1, 2) gebildetem Kallus zu amalgamieren und zu kompaktieren.

8. Vorrichtung nach Patentanspruch 7, **dadurch gekennzeichnet,** dass das genannte Material mit den knochenintegrierenden Eigenschaften ein bio-keramisches Material ist.

9. Vorrichtung nach Patentanspruch 8, **dadurch gekennzeichnet**, dass das genannte bio-keramische Material ein poröses und durchlässiges Material ist.

10. Vorrichtung nach einem beliebigen der vorstehenden Patentansprüche, **dadurch gekennzeichnet,** dass das Rohr (3) aus wenigstens zwei Längshälften (17, 18) besteht, von denen eine jede Verbindungsmittel (13) hat, wobei diese Hälften körperverträglich und dazu bestimmt sind, ein stabiles Zusammensetzen des Rohres (3) zu gewährleisten.

11. Vorrichtung nach einem beliebigen der vorstehenden Patentansprüche, **dadurch gekennzeichnet,** dass die innere Oberfläche (3c) des Rohres (3), welche die Kammer (C) begrenzt, mit einer Schicht (14) aus körperverträglichem und bio-resorbierbaren Material ausgekleidet ist, welche mit langsam freigebenden Substanzen geladen werden kann, wodurch die Reproduktion des Kallus begünstigt wird.

12. Vorrichtung nach einem beliebigen der vorstehenden Patentansprüche, **dadurch gekennzeichnet,** dass die innere Oberfläche (3c) des Rohres (3), welche die Kammer (C) begrenzt, mit einer Schicht aus körperverträglichem und bio-resorbierbaren Material ausgekleidet ist, wobei die genannte Schicht (14) dazu bestimmt ist, einen Sitz für Zellkulturen zu bilden, wie Knochenmarkzellen und Knochenbilder, welche die Reproduktion des Kallus fördern.

13. Vorrichtung nach einem beliebigen der vorstehenden Patentansprüche, **dadurch gekennzeichnet**, dass das Rohr (3) die Form eines Zylinders oder eines Stumpfkegels hat.

14. Vorrichtung nach einem beliebigen der vorstehenden Patentansprüche, **dadurch gekennzeichnet,** dass das Rohr (3) eine Kammer (C) beschreibt, wobei die genannte Kammer (C) im wesentlichen abgedichtet ist.

## Revendications

1. Une prothèse orthopédique, applicable notamment sur au moins deux fragments d'os, proximal (1) et distal (2), dérivant d'une fracture d'un segment osseux (12), lesdits fragments (1,2) étant placés avec leurs têtes respectives (1a, 2a) l'une en face de l'autre, où ladite prothèse orthopédique comporte un tube (3), fabriqué dans une matière biocompatible et biorésorbable, pouvant être introduit entre ledit couple de fragments (1, 2), assure la contention, à ses deux extrémités opposées (3a, 3b), des têtes (1a, 2a) des fragments d'os (1, 2) et est dotée de systèmes (4), en matière biocompatible et biorésorbable, pour la fixation des têtes (1a, 2a) sur les fragments d'os (1, 2) ; ladite prothèse orthopédique étant caractérisée en ce que lesdits fragments d'os (1, 2) sont positionnés avec leurs têtes respectives (1a, 2a) à une distance (D) suite à une perte de matière osseuse dudit segment osseux (12) qui empêche la remise en place correcte dudit segment (12), en tirant ensemble et en créant un contact substantiel entre les têtes (1a, 2a) des fragments correspondants (1, 2), avec lesdits systèmes de fixation (4) placés aux extrémités (3a, 3b) du tube (3), et en ce que la forme et la structure du tube (3) sont telles qu'elles définissent, au moins sur toute la distance (D) séparant les deux fragments (1, 2), une chambre (C) pour la protection, la contention, le guidage et la régénération du cal des têtes (1a, 2a) desdits fragments (1, 2) du segment osseux (12), afin de se lier avec le cal et devenir une partie intégrale de celui-ci, et en ce que la paroi du tube (3) est continue sur sa périphérie, sauf au niveau des trous débouchants (8) et des systèmes (5) de liaison à la sortie et/ou l'entrée.

2. La prothèse orthopédique selon la revendication 1, caractérisée en ce que le tube (3) est fabriqué dans une matière biorésorbable ayant des propriétés physiques et chimiques qui garantissent une résorption contrôlée dans le temps, dépendant du temps nécessaire à la régénération du cal entre les deux fragments d'os (1, 2).

3. La prothèse orthopédique selon la revendication 1, caractérisée en ce que le tube (3) est doté d'au moins deux systèmes (5) pour relier ladite chambre (C) avec l'environnement extérieur, afin de permettre l'introduction, à l'intérieur de ladite chambre (C), de substances pharmaceutiques et/ou biologiques.

4. La prothèse orthopédique selon la revendication 3, caractérisée en ce que lesdits systèmes (5) de liaison avec l'environnement extérieur consistent en au moins un conduit (6) ou cathéter, dont une extrémité (6a) se trouve à l'intérieur du tube (3) et l'autre extrémité (6b) débouche à l'extérieur du segment osseux (12).

5. La prothèse orthopédique selon la revendication 3, caractérisée en ce que lesdits systèmes (5) de liaison de la chambre (C) avec l'environnement extérieur comprend un autre conduit de sortie (15), dont une extrémité (15a) se trouve à l'intérieur du tube (3) et l'autre extrémité (15b) débouche à l'extérieur du segment osseux (12).

6. La prothèse orthopédique selon la revendication 1, caractérisée en ce que le tube (3) est de préférence fabriqué à partir d'un polymère.

7. La prothèse orthopédique selon la revendication 1, caractérisée en ce que le tube (3) est fabriqué dans une matière biocompatible ayant des propriétés physiques et chimiques d'intégration osseuse et étant conçu afin de s'amalgamer et de se compacter avec le cal généré entre les deux têtes (1a,2a ) des fragments d'os (1, 2).

8. La prothèse orthopédique selon la revendication 7, caractérisée en ce que ladite matière dotée de propriétés d'intégration avec l'os est une matière biocéramique.

9. La prothèse orthopédique selon la revendication 8, caractérisée en ce que ladite matière biocéramique est poreuse et perméable.

10. La prothèse orthopédique selon l'une quelconque des revendications précédentes, caractérisée en ce que le tube (3) comporte au moins deux moitiés longitudinales (17, 18), dotées chacune d'éléments de liaison (13), où lesdites moitiés sont biocompatibles et conçues afin de garantir un assemblage stable du tube (3).

11. La prothèse orthopédique selon l'une quelconque des revendications précédentes, caractérisée en ce que la surface interne (3c) du tube (3) délimitant la chambre (C) est revêtue d'une couche (14) de matière biocompatible et biorésorbable, qui peut être chargée à l'aide de substances à libération lente favorisant la reproduction du cal.

12. La prothèse orthopédique selon l'une quelconque des revendications précédentes, caractérisée en ce que la surface interne (3c) du tube (3) délimitant la chambre (C) est revêtue d'une couche (14) de matière biocompatible et biorésorbable, ladite couche (14) étant conçue pour former le site de cultures cellulaires, telles que des cellules de moelle osseuse et des ostéoblastes qui favorisent la reproduction du cal.

13. La prothèse orthopédique selon l'une quelconque des revendications précédentes, caractérisée en ce que le tube (3) a la forme d'un cylindre ou d'un cône tronqué.

14. La prothèse orthopédique selon l'une quelconque des revendications précédentes, caractérisée en ce que le tube (3) définit une chambre (C), ladite chambre (C) étant essentiellement hermétique.
